(19) Europäisches Patentamt
European Patent Office
Office européen des brevets

(11) Publication number: 0 431 934 A2

# (12) EUROPEAN PATENT APPLICATION

(21) Application number: 90313241.3

(51) Int. Cl.[5]: C07C 69/22, C09K 19/32

(22) Date of filing: 06.12.90

(30) Priority: 06.12.89 IT 2264189

(43) Date of publication of application: 12.06.91 Bulletin 91/24

(84) Designated Contracting States: AT BE CH DE DK ES FR GB GR LI NL SE

(71) Applicant: MONTEDIPE S.r.l.
31, Foro Buonaparte
I-20121 Milan (IT)

(72) Inventor: Dalcanale, Enrico
26, Via Turbigo
I-28067 Pernate, Novara (IT)
Inventor: Bonsignore, Stefanio
8, Via Bergamo
I-28100 Novara (IT)
Inventor: Cometti, Giuseppe
14, Via Crocetta
I-28048 Verbania Pallanza, Novara (IT)
Inventor: Guglielmetti, Gianfranco
15 A, Via IV Novembre
I-28010 Bogogno, Novara (IT)
Inventor: Du Vosel, Annick
Cascina Molinaccio
I-28010 Caltignaga, Novara (IT)

(74) Representative: Jones, Helen Marjorie Meredith et al
Gill Jennings & Every 53-64 Chancery Lane
London WC2A 1HN (GB)

## (54) Mixtures of pyramidal columnar liquid crystals and procedure for their preparation.

(57) Mixtures of pyramidal columnar liquid crystals made up of dodeca-substituted derivatives of macrocyclic tetramers wherein each of the substituents is either linear or branched alkanoyl chain of different lengths and randomly distributed. These mixtures show stable mesophases of the columnar type having a wide temperature range which also includes room temperature.

EP 0 431 934 A2

Jouve, 18, rue Saint-Denis, 75001 PARIS

# MIXTURES OF PYRAMIDAL COLUMNAR LIQUID CRYSTALS AND PROCEDURE FOR THEIR PREPARATION

## FIELD OF THE INVENTION

The present invention concerns mixtures of pyramidal columnar liquid crystals.

More precisely the present invention concerns liquid crystals made up of mixtures of dodeca-substituted macrocyclic tetramers wherein the mesophases are of the pyramidal columnar type.

## BACKGROUND OF THE INVENTION

Thermotropic macrocyclic tetramers showing mesophases of the pyramidal columnar type are described in the Italian patent application No. 21308 A/88.

However each of these pure products, which have been obtained by means of esterification with acyl halides of the tetramers produced by the acid-catalysed condensation of pyrogallol and an acetal of an alkyl aldehyde, has a maximum mesophase range of about 25°C which is not sufficient for application in the optoelectronic field.

Moreover, the stability of some of these mesophases, especially those related to homologous products having a short alkanoyl chain, has a limited duration.

On the other hand, mixtures of these pure products having different molar percentages do not show mesophases with temperature ranges which are significantly wide.

## SUMMARY OF THE INVENTION

The Applicant has now discovered that mixtures of dodeca-substituted derivatives of macrocyclic tetramers, in which the randomly distributed substituents are alkanoyl chains of different lengths, show mesophases of columnar type which are indefinitely stable with a very wide temperature range.

Consequently the subject of the present invention concerns mixtures of pyramidal columnar liquid crystals obtained from the reaction of a macrocyclic tetramer having the general formula :

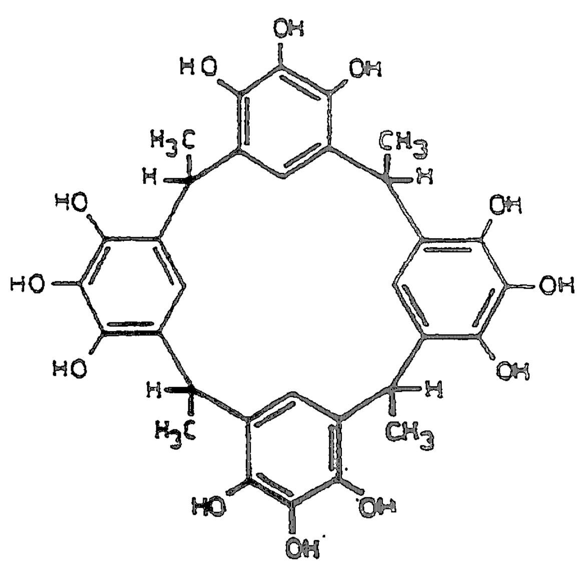

(I)

with a mixture of acyl halides R-X in which X stands for a halogen atom such as chlorine or bromine, and R for a group

—COR′ (II)

where R′ is an alkyl radical, either linear or branched, with a number of carbon atoms higher than or equal to 5, generally between 5 and 25, and in which mixture at least two halides have a different number of carbon atoms.

## DESCRIPTION OF THE INVENTION

The products described in the present invention are consequently made up of mixtures of macrocyclic tetramers having the general formula :

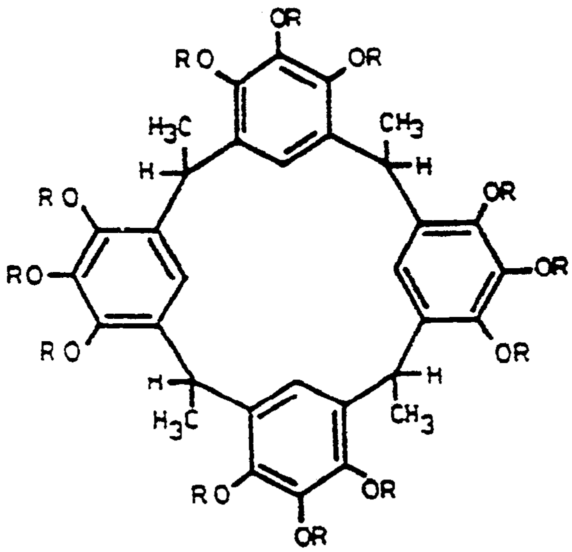

(III)

in which at least two R groups are different from each other.

In the thermotropic macrocyclic tetramers referred to in the present invention, the $CH_3$ groups are arranged in an axial position as shown in Fig. 1 which gives a perspective view of the molecule. This kind of structure tends to be set out in the mesophase in a hexagonal lattice in the form of a columnar arrangement.

The melting points of the mixtures of tetramers having the general formula (III) range between -30 and +50°C. The clearing points of the molten products range between -15 and +70°C.

The pyramidal columnar liquid crystalline arrangement as shown by analyses using a Differential Scanning Calorimeter (DSC) and a polarized light optical microscope, and using mixtures with pyramidal columnar liquid crystal a having a known $D_{ho}$ structure.

All these observations concur in defining the mesophase as hexagonal of the ($D_{ho}$) type.

The mixtures of thermotropic macrocyclic tetramers referred to in the present invention are prepared by the reaction between the macrocyclic tetramers having formula (I) with mixtures of acyl halides having the formula R-X where R and X have the above mentioned meaning.

These mixtures of halides may be composed of N components where N ranges between 2 and 10, preferably between 2 and 4. The molar percentage of each of the acyl halides of which the reagent mixture is composed may vary between a minimum of 5% and a maximum of 95%.

The tetramer having the general formula (I) is obtained by the acid-catalysed condensation at room temperature of pyrogallol and an aldehyde which term includes acetals, in a solvent such as methanol or ethanol, where the catalyst may be selected between hydrochloric acid, sulfuric acid, phosphoric acid, para-toluenesulfonic acid, etc.. ; these catalysts are used in amounts of between 1 and 20% of the total amount of the reagents.

More specifically, the tetramer having the general formula (I) is prepared in accordance with the following reaction scheme :

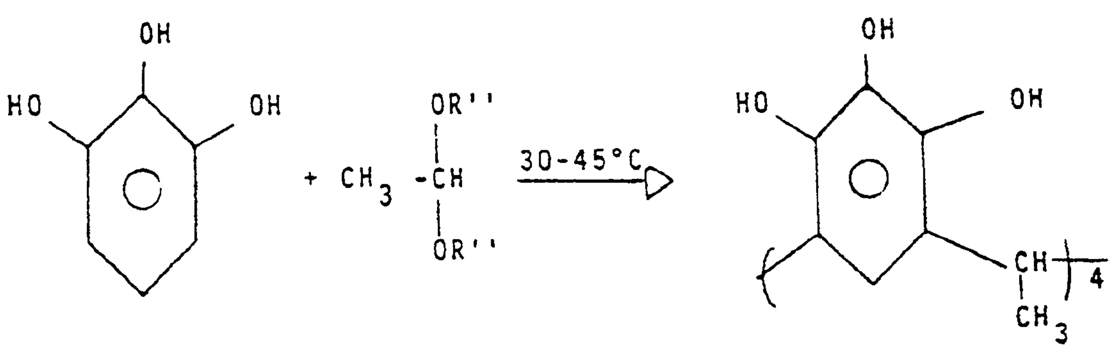


where R" is an alkyl, isoalkyl, cycloalkyl, aryl, alkylaryl radical containing from 1 to 30 carbon atoms.

Examples of aldehydes which can be used are 1,1-diethoxyethane, 1,1-dipropoxyethane, 1,1-dicyclohexyloxyethane, 1,1-dibenzoxyethane, etc..

The product thus obtained is made to react with the acyl halides R-X.

Examples of acyl halides are 2-ethyl hexanoyl chloride, hexanoyl chloride, heptanoyl chloride, octanoyl chloride, 4-methyl nonanoyl chloride, decanoyl chloride, nonanoyl chloride, tridecanoyl chloride, dodecanoyl chloride, tetradecanoyl chloride, hexadecanoyl chloride, octadecanoyl chloride, etc...

The reaction is carried out as a bulk esterification under normal pressure and at a temperature ranging between +50 and +200°C.

The number and molar percentage of the tetramers which make up the mixture depends on the number and total percentage of the acyl halides of which the reagent mixture is composed.
For a mixture of tetramers from two acyl halides A and B at a 1/1 molar ratio the theoretical distribution is represented by a Gauss curve as shown in Fig. 2 where the compositions are plotted against the percentages of the distributions.

By varying the number of the components in the mixture of the acyl halides and/or their molar ratio, it is thus possible to obtain mixtures of liquid crystals having an indefinite stability and a wide thermal range of mesophase. More specifically, by varying the length of the alkanoyl chains, it is possible to obtain stable mesophases at low temperatures (short chain) or at high temperatures (long chains). It should be noted that equally dodeca-substituted tetramers having alkanoyl chains with a number of carbon atoms which is lower than 11 or higher than 18, if considered individually, are not liquid crystals. It is therefore possible to prepare mixtures of liquid crystals for the application in the optoelectronic field in accordance with specific requirements of use. In fact, as it can be seen from the following examples, the maximum limit of the transition temperature of the mesophase-isotrope phase is a function of the length and molar percentage in the reagent mixture of the longest alkanoyl chain, whereas the transition temperature of the crystalline phase-mesophase depends on the type, length and molar percentage of the shortest alkanoyl chain.

To enable a clearer interpretation and to allow the practical enforcement of the present invention, the following examples are listed below as an illustration of said invention without limiting it in any way.

EXAMPLE 1

Preparation of the tetramer having the formula (I). 12.61 g (0.1 M) of pyrogallol are dissolved, in an argon atmosphere, in 50 ml of ethanol and 10 ml of a 37% solution of HCl. The solution is cooled by means of an ice-bath to 0°C and 14.22 ml of 1,1-diethoxyethane are dripped into it over a period of an hour. The solution is kept at room temperature under agitation for 48 hours and is then heated under reflux for 6 hours. It is then cooled to room temperature, water is added and the precipitate thus obtained is filtered and is repeatedly rinsed with water until it becomes neutral. The product is dried under vacuum ($1 \times 10^{-3}$ torr/25°C). 11.0 g of pure product (yield 72%) are obtained.
Mass ($DCI^+$) : $MH^+$ = 609.
$^1H$ - NMR (DMSO-$d_6$) :

$\delta$ 1.55 (d, 12H, J = 7.5 Hz, $CH_3$) ; 4.56 (q, 4H, J = 7.5 Hz, CH) ; 6.79 (s, 4H, Ar-H) ; 8.02 (bs, 4H, OH) ; 8.22 (s, 8H, OH).
Elemental analysis for $C_{32}H_{32}O_{32}$ :
- Theoretical : C = 63.15% H = 5.30%
- Experimental : C = 63.01% H = 5.43%

EXAMPLE 2

Preparation of the mixture of dodeca-substituted tetramers obtained by a reaction between the tetramer having the formula (I) and a homogeneous mixture made up of octanoyl chloride and tetradecanoyl chloride at a molar ratio of 1/1.

0.609 g (1 mM) of tetramer having formula (I) are added to a homogeneous mixture made up of 4.88 g (30 mM) of octanoyl chloride and 7.0 g (30 mM) of tetradecanoyl chloride. The whole mixture is heated to 160°C for 16 hours. When the reaction has finished, the excess chlorides are distilled under vacuum at $10^{-2}$ torr. The remaining residue is dissolved in methylene chloride and the solution obtained is first extracted with NaOH 0.2 N, washed with water until neutral and finally dried on sodium sulphate. The solvent is evaporated and the crude residue is purified on a silica gel column using methylene chloride/hexane 4/1 as an eluent. 0.96 g of the pure

product, having the following composition (DCI-MS positive ions), are obtained : Theoretical interval : 2120 (12 $C_8$) - 3128 (12 $C_{14}$). Experimental interval : 2288 (10 $C_8$ - 2 $C_{14}$, 5%) ; 2372 (9 $C_8$ - 3 $C_{14}$, 10%) ; 2456 (8 $C_8$ - 4 $C_{14}$, 35%) ; 2540 (7 $C_8$ - 5 $C_{14}$, 50%) ; 2624 (6 $C_8$ - 6 $C_{14}$, 80%) ; 2708 (5 $C_8$ - 7 $C_{14}$, 100%) ; 2792 (4 $C_8$ - 8 $C_{14}$, 85%) ; 2876 (3 $C_8$ - 9 $C_{14}$, 40%) ; 2960 (2 $C_8$ - 10 $C_{14}$, 15%) ; 3044 (1 $C_8$ - 11 $C_{14}$, 5%).

EXAMPLE 3

With the same procedure as in Example 2 and using hexanoyl chloride and tetradecanoyl chloride at a ratio of 1/1, the following mixture (0.83 g) was obtained : Theoretical interval : 1784 (12 $C_6$) - 3128 (12 $C_{14}$). Experimental interval : 2232 (8 $C_6$ - 4 $C_{14}$, 5%) ; 2344 (7 $C_6$ - 5 $C_{14}$, 40%) ; 2456 (6 $C_6$ - 6 $C_{14}$, 95%) ; 2568 (5 $C_6$ - 7 $C_{14}$, 100%) ; 2680 (4 $C_6$ - 8 $C_{14}$, 90%) ; 2792 (3 $C_6$ - 9 $C_{14}$, 50%) ; 2904 (2 $C_6$ - 10 $C_{14}$, 15%) ; 3016 (1 $C_6$ - 11 $C_{14}$, 5%).

EXAMPLE 4

With the same procedure as in Example 2 and using 4-methyl nonanoyl chloride and tetradecanoyl chloride at a molar ratio of 1/1, the following mixture (0.33 g) was obtained after purification on a silica gel column using methylene chloride/hexane 3/1 as an eluent : Theoretical interval : 2456 (12 $C_{10iso}$) - 3128 (12 $C_{14}$). Experimental interval : 2512 (11 $C_{10iso}$ - 1 $C_{14}$, 5%) ; 2568 (10 $C_{10iso}$ - 10 $C_{14}$, 10%) ; 2624 (9 $C_{10iso}$ - 3 $C_{14}$, 20%); 2680 (8 $C_{10iso}$ - 4 $C_{14}$, 40%) ; 2736 (7 $C_{10iso}$ - 5 $C_{14}$, 70%) ; 2792 (6 $C_{10iso}$ - 6 $C_{14}$, 100%) ; 2848 (5 $C_{10iso}$ - 7 $C_{14}$, 70%) ; 2904 (4 $C_{10iso}$ - 8 $C_{14}$, 50%) ; 2960 (3 $C_{10iso}$ - 9 $C_{14}$, 15%) ; 3016 (2 $C_{10iso}$ - 10 $C_{14}$, 10%).

EXAMPLE 5

With the same procedure as in Example 2 and using 2-ethyl hexanoyl chloride and hexadecanoyl chloride at a molar ratio of 1/3, the following mixture (0.86 g) was obtained : Theoretical interval : 2120 (12 $C_{8iso}$) - 3464 (12$C_{16}$). Experimental interval : 3128 (3 $C_{8iso}$ - 9 $C_{16}$, 15%) ; 3240 (2 $C_{8iso}$ - 10 $C_{16}$, 40%) ; 3352 (1 $C_{8iso}$ - 11$C_{16}$, 100%); 3464 (12$C_{16}$).

EXAMPLE 6

With the same procedure as in Example 2 and using tetradecanoyl chloride and octadecanoyl chloride at a molar ratio of 1/1, the following mixture (2.11 g) was obtained : Theoretical interval : 3128 (12 $C_{14}$) - 3800 (12$C_{18}$). Experimental interval : 3184 (11 $C_{14}$ - 1 $C_{18}$, 10%) ; 3240 (10 $C_{14}$ - 2 $C_{18}$, 20%) ; 3296 (9 $C_{14}$ - 3 $C_{18}$, 50%) ; 3352 (8 $C_{14}$ - 4 $C_{18}$, 70%) ; 3408 (7 $C_{14}$ - 5 $C_{18}$, 95%) ; 3464 (6 $C_{14}$ - 6 $C_{18}$, 100%) ; 3520 (5 $C_{14}$ - 7 $C_{18}$, 55%); 3576 (4 $C_{14}$ - 8 $C_{18}$, 40%) ; 3632 (3 $C_{14}$ - 9 $C_{18}$, 20%) ; 3688 (2 $C_{14}$ - 10 $C_{18}$, 5%).

EXAMPLE 7

With the same procedure as in Example 2 and using dodecanoyl chloride and octadecanoyl chloride at a molar ratio of 2/3, the following mixture (1.64 g) was obtained : Theoretical interval : 2792 (12 $C_{12}$) - 3800 (12 $C_{18}$). Experimental interval : 3212 (7 $C_{12}$ - 5 $C_{18}$, 5%) ; 3296 (6 $C_{12}$ - 6 $C_{18}$, 35%) ; 3380 (5 $C_{12}$ - 7 $C_{18}$, 75%) ; 3464 (4 $C_{12}$ - 8 $C_{18}$, 100%) ; 3548 (3 $C_{12}$ - 9 $C_{18}$, 85%) ; 3632 (2 $C_{12}$ - 10 $C_{18}$, 60%) ; 3716 (1 $C_{12}$ - 11 $C_{18}$, 20%).

EXAMPLE 8

With the same procedure as in Example 2 and using octanoyl chloride and octadecanoyl chloride at a molar ratio of 1/1, the following mixture (1.18 g) was obtained : Theoretical interval : 2120 (12 $C_8$) - 3800 (12 $C_{18}$). Experimental interval : 2540 (9 $C_8$ - 3 $C_{18}$, 15%) ; 2680 (8 $C_8$ - 4 $C_{18}$, 20%) ; 2820 (7 $C_8$ - 5 $C_{18}$, 40%) ; 2960 (6 $C_8$ - 6 $C_{18}$, 75%) ; 3100 (5 $C_8$ - 6 $C_{18}$, 100%) ; 3240 (4 $C_8$ - 8 $C_{18}$, 65%) ; 3380 (3 $C_8$ - 9 $C_{18}$, 15%).

EXAMPLE 9

With the same procedure as in Example 2 and using tetradecanoyl chloride, hexadecanoyl choride and octadecanoyl chloride at a molar ratio of 1/1/1, the following mixture was obtained :
Theoretical interval : 3128 (12 $C_{14}$) - 3800 (12 $C_{18}$).
Experimental interval : 3296 (35%) ; 3324 (40%) ; 3352 (50%) ; 3380 (75%) ; 3408 (80%) ; 3436 (100%) ; 3464 (80%) ; 3492 (75%) ; 3520 (50%) ; 3548 (30%) ; 3576 (15%) ; 3604 (10%) ; 3632 (5%).
Table 1 shows the transition temperatures of the synthesized mixtures compared to those of certain single products.

TABLE 1

Transition temperatures ($^0C$) for the mixtures of tetramers in Examples 1-8 compared to those of certain single products.

| Example | $R_1$ | $R_2$ | $R_3$ | Molar ratio | K | | C | | I |
|---|---|---|---|---|---|---|---|---|---|
| - | $C_{14}$ | - | - | - | ■ | 46 | ■ | 65.5 | ■ |
| 1 | $C_{14}$ | $C_8$ | - | 1/1 | ■ | -10 | ■ | 40 | ■ |
| 2 | $C_{14}$ | $C_6$ | - | 1/1 | ■ | | | 42.5 | ■ |
| 3 | $C_{14}$ | $C_{10iso}$ | - | 1/1 | | | | -15 | ■ |
| - | $C_{16}$ | - | - | - | ■ | 48 | ■ | 61 | ■ |
| 4 | $C_{16}$ | $C_{8iso}$ | - | 3/1 | ■ | 33 | ■ | 55 | ■ |
| - | $C_{18}$ | - | - | - | ■ | 58 | ■ | 68 | ■ |
| 5 | $C_{18}$ | $C_{14}$ | - | 1/1 | ■ | 41 | ■ | 64 | ■ |
| 6 | $C_{18}$ | $C_{12}$ | - | 3/2 | ■ | 36 | ■ | 51.5 | ■ |
| 7 | $C_{18}$ | $C_8$ | - | 1/1 | ■ | <-20 | ■ | 40.5 | ■ |
| 8 | $C_{18}$ | $C_{16}$ | $C_{14}$ | 1/1/1 | ■ | 40.5 | ■ | 65.5 | ■ |

K = crystalline phase

C = columnar liquid crystalline phase

I = isotrope phase

R1, R2, R3 = radicals of chloride products

EXAMPLE 10 (Comparative example)

A mixture was obtained by combining at an equal molar ratio two tetramers having the general formula III, each one with equal alkanoyl chains containing respectively 18 and 10 carbon atoms and at a temperature higher than the clearing point of both. Table 2 shows the phase transition temperatures.

EXAMPLES 11-16 (Comparative examples)

Table 2 shows the transition temperatures for the mixtures obtained by combining, at the molar rations indicated, macrocyclic tetramers substituted with alkanoyl chains having a number of carbon atoms as shown in the table and using the procedure described in Example 9.

TABLE 2

| Example | Mixture | Molar ratio | K | | C | | I |
|---|---|---|---|---|---|---|---|
| 9 | $C_{18}/C_{10}$ | 1/1 | ■ | | | 55 | ■ |
| 10 | $C_{18}/C_{12}$ | 1/1 | ■ | 52 | ■ | 55 | ■ |
| 11 | $C_{18}/C_{14}$ | 1/1 | ■ | 50,5 | ■ | 63,5 | ■ |
| 12 | $C_{18}/C_{16}$ | 1/4 | ■ | 53 | ■ | 68 | ■ |
| 13 | $C_{18}/C_{16}$ | 1/1 | ■ | 56,5 | ■ | 67 | ■ |
| 14 | $C_{18}/C_{16}$ | 4/1 | ■ | 58,5 | ■ | 68 | ■ |
| 15 | $C_{18}/C_{16}/C_{14}$ | 1/1/1 | ■ | 48.5 | ■ | 66 | ■ |

## Claims

1. Mixtures comprising macrocyclic tetramers having the general formula :

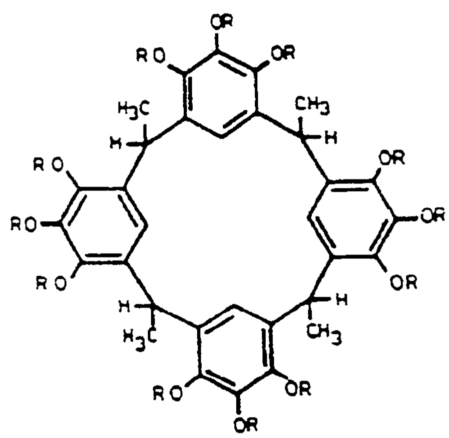

(III)

in which at least two of the R groups are different from each other.

2. Mixtures of pyramidal columnar liquid crystals obtainable by the reaction of a macrocyclic tetramer having the general formula :

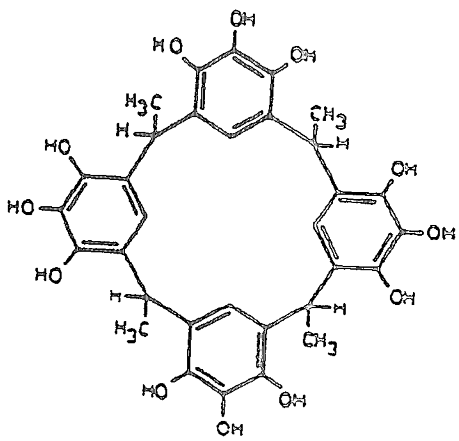

(I)

with a mixture of acyl halides R-X where X stands for a halogen atom such as chlorine or bromine, and R stands for a group

—COR′ (II)

where R′ is a linear or branched alkyl radical, with a number of carbon atoms higher than or equal to 5 and in which mixture at least two halides have a differing number of carbon atoms.

3. A mixture according to claim 2 in which R′ is a linear or branched alkyl radical with between 5 and 25 carbon atoms.

4. Mixtures in accordance with any preceding claim, having melting points which range between -30 and +50°C and clearing points of the molten products of between -15 and +70°C.

5. A process for preparation of mixtures of pyramidal columnar liquid crystals by reaction of a macrocyclic tetramer having the general formula :

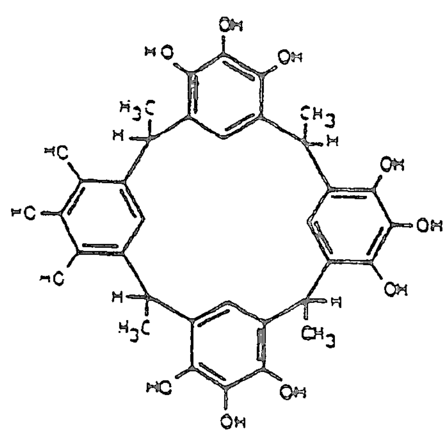

(I)

by reacting with a mixture of acyl halides R-X where X stands for a halogen atom such as chlorine or bromine, and R stands for a group

—COR′ (II)

where R′ is a linear or branched alkyl radical, with a number of carbon atoms higher than or equal to 5, and in which mixture at least two halides have a differeing number of carbon atoms.

6. A process according to claim 5 in which R′ is a linear or branched alkyl radical with between 5 and 25 carbon atoms.

7. A process in accordance with claim 5 or claim 6 in which the mixtures of halides have N components where N ranges between 2 and 10, preferably between 2 and 4.

8. A process in accordance with any of claims 5 to 7 in which each of the acyl halides which make up the

reagent mixture may vary between a minimum of 5% and a maximum of 95%.

**9.** A process in accordance with any of claims 5 to 8 in which the reaction is carried out as a bulk esterification reaction under normal pressure and at a temperature between +50°C and +200°C.

**10.** Use of the mixtures of columnar liquid crystals in accordance with any of claims 1 to 4 for application in the optoelectronic field.

**11.** Use of the mixtures of columnar liquid crystals produced by the process of any of claims 5 to 9 for application in the optoelectronic field.

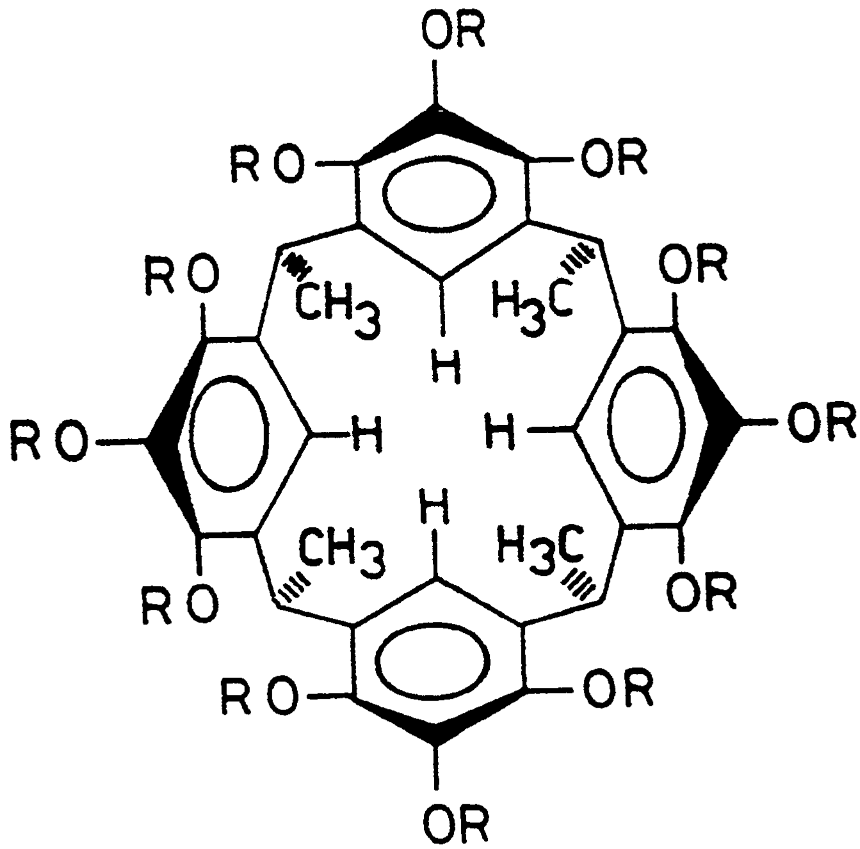

Fig.1

Fig.2

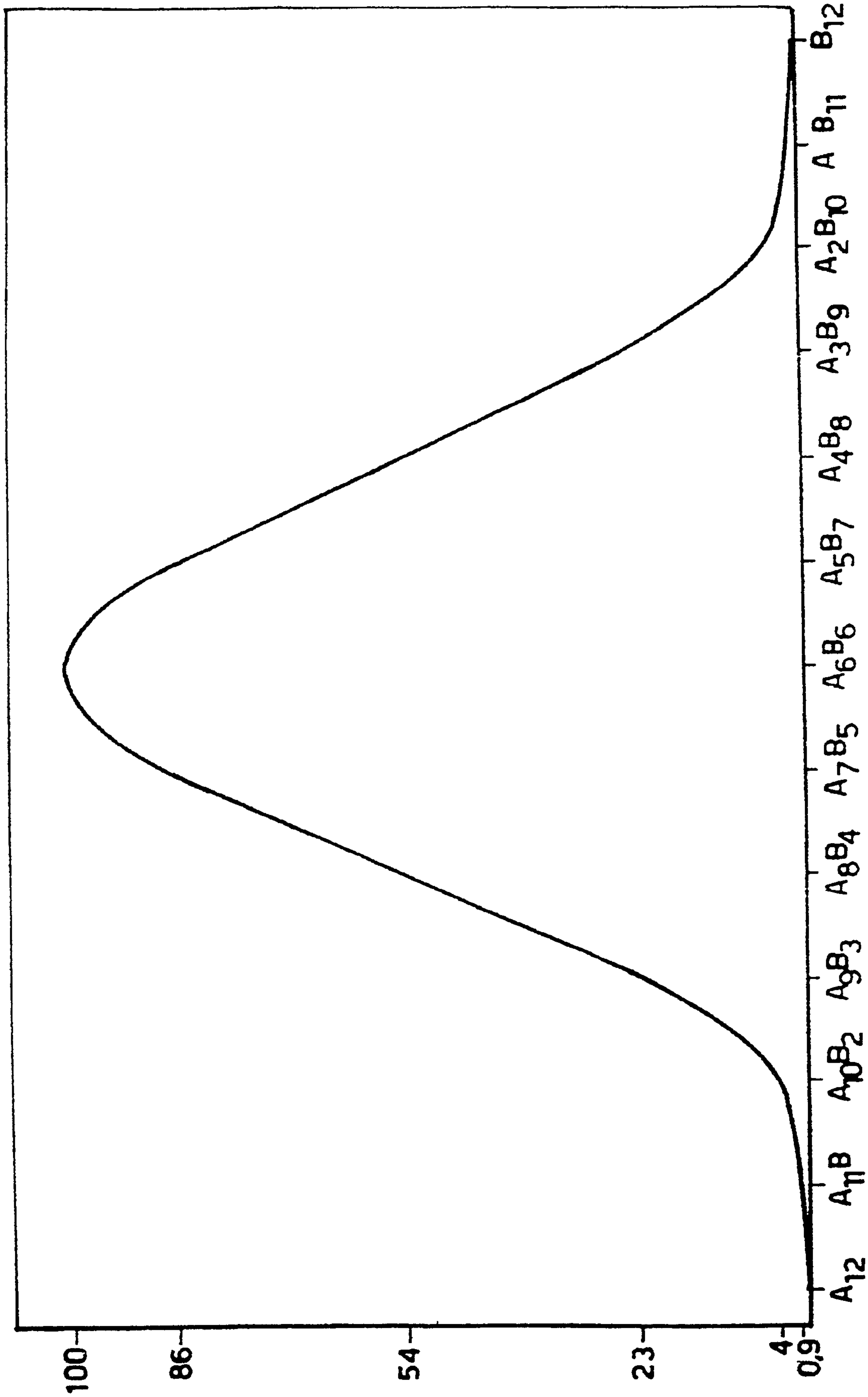
100
86
54
23
4
0,9
A12
A11B
A10B2
A9B3
A8B4
A7B5
A6B6
A5B7
A4B8
A3B9
A2B10
A B11
B12